# EUROPEAN PATENT APPLICATION

(11) **EP 3 195 993 A1**
(43) Date of publication of application: **26.07.2017**
(21) Application number: 16755051.6
(22) Date of filing: 12.01.2016
(51) Int. Cl.: B25J 17/00, A61B 90/00, B25J 1/02

(54) **MANIPULATOR AND MANIPULATOR SYSTEM**

(30) Priority: 25.02.2015 JP 2015035323
(71) Applicant: OLYMPUS CORPORATION, Hachioji-shi Tokyo 192-8507 (JP)
(72) Inventor: YOSHIMURA, Katsuhiko, Tokyo 192-8507 (JP)
(74) Representative: Schicker, Silvia
(86) International application number: PCT/JP2016/050676
(87) International publication number: WO 2016/136301

(57) **Abstract**

The invention has for its object to provide a manipulator and a manipulator system that enables a bending assembly to be unerringly regulated even under abnormal conditions where power of a drive unit is not transmitted to the bending assembly.

The manipulator 1 includes a main unit 2, a elongated member 3 that extends from the main unit 2, a bending assembly 4 connected to the elongated member 3, a coupling member 5 for transmission of power for putting the bending assembly 4 into operation, a drive unit 21 for generating power for the coupling member 5, and a regulator 6 that is interposed between the bending assembly 4 and the drive unit 21 and urges the coupling member 5 in a direction of intersecting a path taken by the coupling member 5.

## Description

### Technical Field

The present invention relates to a manipulator, and a manipulator system that can be so bent by bending of a joint assembly that a variety of treatments can be carried out.

### Background Art

Typically, there has been a manipulator widely used in which while a treatment tool is inserted through the patient's body cavity, the distal end of the treatment tool is pulled as by a wire so that it is bent to observe, and apply treatments, to organs in the body cavity. Often during surgical operations, a plurality of treatment tools such as an endoscope for observations, forceps that grasp tissues in the body and an electric scalpel for excising off tissues are inserted through the body cavity.

With such a manipulator so far in the art, there has been a possibility that the bending assembly inserted through the body cavity may be fixed while remaining bent and inoperable by reason of power supply shutdown, a motor breakdown or the like.

To avoid this, Patent Literature 1 has proposed to mount a handle to an electromagnetic clutch to allow for manual operation even after a release of transmission of power from a motor.

### Citation List

### Patent Literature

Patent Literature 1: Japan Patent No. 4722245

### Summary of Invention

### Technical Problem

For manual operation of the manipulator disclosed in Patent Literature 1, however, it is required to cut off transmission of driving power between the motor and the gear as by a clutch mechanism, incompatible with a malfunction in a cutoff mechanism. The manipulator is also not well compatible with a breakdown of the driver or power transmission such as gear engagement.

With the aforesaid problems in mind, one specific object of the present invention is to provide a manipulator, and a manipulator system that enables a bending assembly to be unerringly regulated even under abnormal conditions where there is no transmission of power from a drive unit to a bending assembly.

### Solution to Problem

According to one embodiment of the invention, there is a manipulator provided, which includes:
a main unit,
a elongated member that extends from the main unit,
a bending assembly connected to the elongated member,
a coupling member for transmission of power for putting the bending assembly into operation,
a drive unit for generating power for the coupling member, and
a regulator that is interposed between the bending assembly and the drive unit and urges the coupling member in a direction of intersecting a path taken by the coupling member.

In the manipulator according to one embodiment of the invention, the regulator includes a moving member that has urged the coupling member in a direction of intersecting the path taken by the coupling member.

In the manipulator according to one embodiment of the invention, the moving member has given an urge to the coupling member.

In the manipulator according to one embodiment of the invention, the coupling member includes a first coupling member that bends the bending assembly in one direction, and a second coupling member that bends the bending assembly in a direction different from a direction in which the bending assembly is bent by the first coupling member.

In the manipulator according to one embodiment of the invention, the moving member includes:
a first moving member that urges the first coupling member in a direction of intersecting a path taken by the first coupling member, and
a second moving member that urges the second coupling member in a direction of intersecting a path taken by the second coupling member, and
the regulator includes a regulating portion that moves the first moving member and the second moving member in one operation in the same direction.

In the manipulator according to one embodiment of the invention,
the first moving member has given an urge to the first coupling member, and
the second moving member has given an urge to the second coupling member in a direction opposite to a direction in which the first coupling member is urged.

In the manipulator according to one embodiment of the invention, the regulator includes a support member designed such that the moving member urges the coupling member thereby bringing the support member in contact with the coupling member from a direction opposite to that of the moving member.

In the manipulator according to one embodiment of the invention, the support member includes:
a first support member designed such that the first moving member urges the first coupling member thereby bringing the first support member in contact with the first coupling member from a direction opposite to that of the first moving member, and
a second support member designed such that the second moving member urges the second coupling member thereby bringing the second support member in contact with the second coupling member from a direction opposite to that of the second moving member.

In the manipulator according to one embodiment of the invention, the support member is rotatable about an axis.

The manipulator according to one embodiment of the invention includes a sheath that covers the coupling member.

In the manipulator according to one embodiment of the invention, the regulator includes:
a casing,
a regulating member supported on the casing,
a resilient member interposed between the casing and the regulating member, and
a stopper that restricts movement of the regulating member relative to the casing.

In the manipulator according to one embodiment of the invention, the stopper can support the regulating member and be removed from the casing.

In the manipulator according to one embodiment of the invention, the stopper is fixed to the casing, and movably supports the regulating member.

The manipulator according to one embodiment of the invention, the stopper includes a disengagement mechanism that supports the regulating member movably relative to the casing.

According to one embodiment of the invention, there is a manipulator system provided, which includes:
the manipulator including a treatment tool and an endoscope at the bending assembly,
an image processor for applying image processing to an image signal obtained through the endoscope, and
a display for displaying an image signal sent out from the image processor.

### Advantageous Effects of Invention

According to some specific embodiments of the invention, it is possible to provide a manipulator, and a manipulator system that allows for unerring regulation or adjustment of a bending assembly even in unusual events where power is not transmitted from the driver to the bending assembly.

### Brief Description of Drawings

Fig. 1 is illustrative of the manipulator according to the first embodiment.
Fig. 2 is illustrative of the regulator in the manipulator according to the first embodiment.
Fig. 3 is illustrative of how the bending assembly in a linear state of the manipulator according to the first embodiment is bent by movement of the regulator.
Fig. 4 is illustrative of how the bending assembly in a bent state of the manipulator according to the first embodiment is made linear by movement of the regulator.
Fig. 5 is illustrative of the regulator in the manipulator according to the second embodiment.
Fig. 6 is illustrative of the regulator in the manipulator according to the third embodiment.
Fig. 7 is illustrative of the regulator in the manipulator according to the fourth embodiment.
Fig. 8 is illustrative of the regulator in the manipulator according to the fifth embodiment.
Fig. 9 is illustrative of the regulator in the manipulator according to the sixth embodiment.
Fig. 10 is illustrative of the regulator in the manipulator according to the sixth embodiment that is kept movable.
Fig. 11 is illustrative of the regulator in the manipulator according to the seventh embodiment.
Fig. 12 is illustrative of another example of the manipulator according to one embodiment described herein.
Fig. 13 is illustrative of yet another example of the manipulator according to one embodiment described herein.
Fig. 14 is illustrative of the manipulator system to which the manipulator according to one embodiment described herein is applied.
Fig. 15 is illustrative in architecture of the manipulator system to which the manipulator according to one embodiment described herein is applied.

### Description of Embodiments

Some embodiments are now explained.

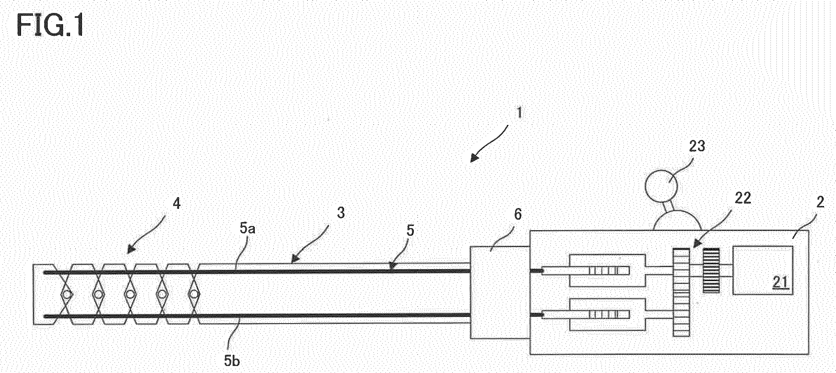

Fig. 1 is illustrative of a manipulator 1 according to the first embodiment.

The manipulator 1 according to the first embodiment includes a main unit 2, a elongated member 3, a bending assembly 4 connected to the elongated member 3, a coupling member 5 such as a wire for transmission of power that puts the bending assembly 4 in operation, and a regulator 6 adapted to regulate the position of the coupling member 5 between the main unit 2 and the bending assembly 4.

The main unit 2 includes a drive unit 21 for generating power for the coupling member 5, a power transmission 22 for transmission of the power generated by the drive unit 21 to the coupling member 5, and a operation unit 23 adapted to put the drive unit 21 into operation. The main unit 2 is formed of or defined by a casing adapted to receive the drive unit 21 and power transmission 22. In the first embodiment, the elongated member 3 extends from the main unit 2 through the regulator 6. On the distal end of the elongated member 3 there is the bending assembly 4 mounted that is bendable or flexible with respect to the elongated member 3. The coupling member 5 includes a first coupling member 5a and a second coupling member 5b, each of which is mounted at one end to the bending assembly 4, inserted through the elongated member 3, and mounted at the other end to the power transmission 2 via the regulator 6.

Usually, the manipulator 1 of such structure puts the operation unit 23 into operation to bend the bending assembly 4. As the operation unit 23 is put into operation, it causes the drive unit 21 to be driven. Power generated by the drive unit 21 gives a pull to the first coupling member 5a or the second coupling member 5b. Upon receiving that pull, the coupling member 5 moves through the elongated member 3 via the regulator 6 to pull one side of the bending assembly 4 and, hence, bend the bending assembly 4.

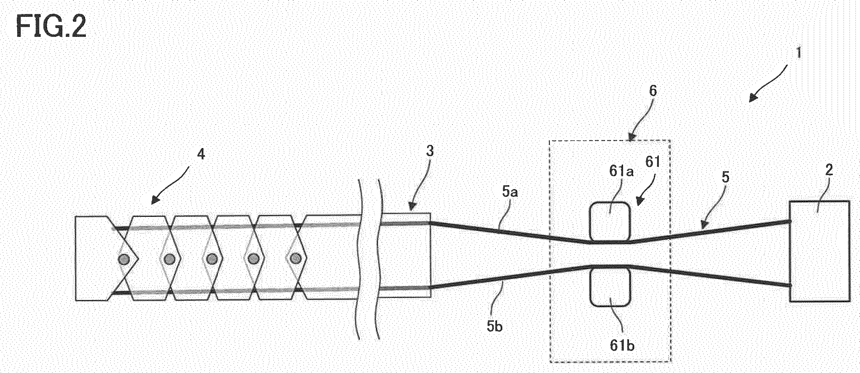

Fig. 2 is illustrative of the regulator 6 in the manipulator 1 according to the first embodiment.

The regulator 6 works if the drive unit 21 is not put into operation by reason of power supply shutdown, a malfunction in the drive unit 21 or the like, or the power of the drive unit 21 is not transmitted to the coupling member 5. In the first embodiment, the regulator 6 is interposed between the main unit 2 and the elongated member 3. The regulator 6 includes a moving member 61 that is movable either by an exclusive driving member or manually and holds two coupling members 5 while they are pressed or urged from both sides. Usually, the coupling member 5 is being urged.

The moving member 61 includes a first moving member 61a that gives an urge to the first coupling member 5a and a second moving member 61b that gives an urge to the second coupling member 5b. The moving member 61 is movable in a direction intersection with the coupling member 5. In particular, the moving member 61 preferably moves in a direction orthogonal to the coupling member 5.

If the bending of the bending assembly 4 can be operated by the operation unit 23, the regulator 6 is not put into operation so that the coupling member 5 can slide over the surface of the regulator 6 to bend the bending assembly 4.

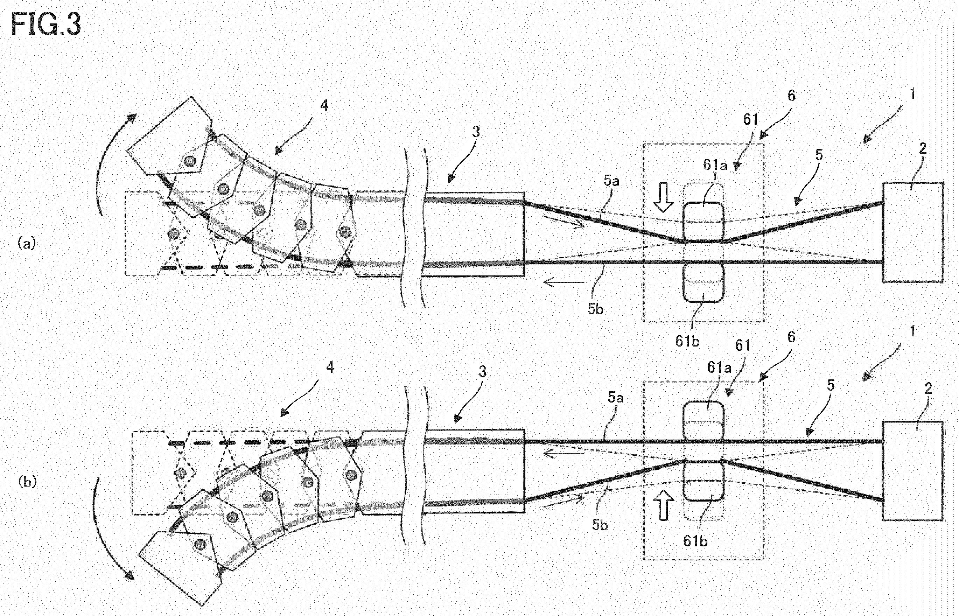

Fig. 3 is illustrative of how the bending assembly 4 in a linear state of the manipulator 1 according to the first embodiment is placed by movement the moving member 61 of the regulator 6 into a bent state: Fig. 3(a) illustrates that an urge is given to the first coupling member 5a and Fig. 3(b) illustrates that an urge is given to the second coupling member 5b.

Referring to Fig. 3(a) where it is difficult to operate the bending assembly 4 in a linear state by the drive unit 21, movement of the moving member 61 of the regulator 6 toward the second coupling member 5b causes a larger urge to be given by the first moving member 61a to the first coupling member 5a so that the second coupling member 5b comes close to a linear state. In other words, the first coupling member 5a for the bending assembly 4 is pulled by the first moving member 61a so that the second coupling member 5b for the bending assembly 4 is relaxed, and the bending assembly 4 is bent toward the first coupling member 5a.

Referring to Fig. 3(b) where it is difficult to operate the bending assembly 4 in a linear state by the drive unit 21, movement of the moving member 61 of the regulator 6 toward the second coupling member 5b causes a larger urge to be given by the second moving member 61b to the first coupling member 5b so that the first coupling member 5a comes close to a linear state. In other words, the second coupling member 5b for the bending assembly 4 is pulled by the second moving member 61b so that the first coupling member 5a for the bending assembly 4 is relaxed, and the bending assembly 4 is bent toward the second coupling member 5b.

Thus, even when the drive unit 21 is not put into operation by reason of power supply shutdown, a malfunction in the drive unit 21 or the like, or even when the power of the drive unit 21 is not transmitted to the coupling member 5, the manipulator 1 according to the first embodiment allows for operation of the moving member 61 of the regulator 6, thereby putting the bending assembly 4 in a linear state into operation.

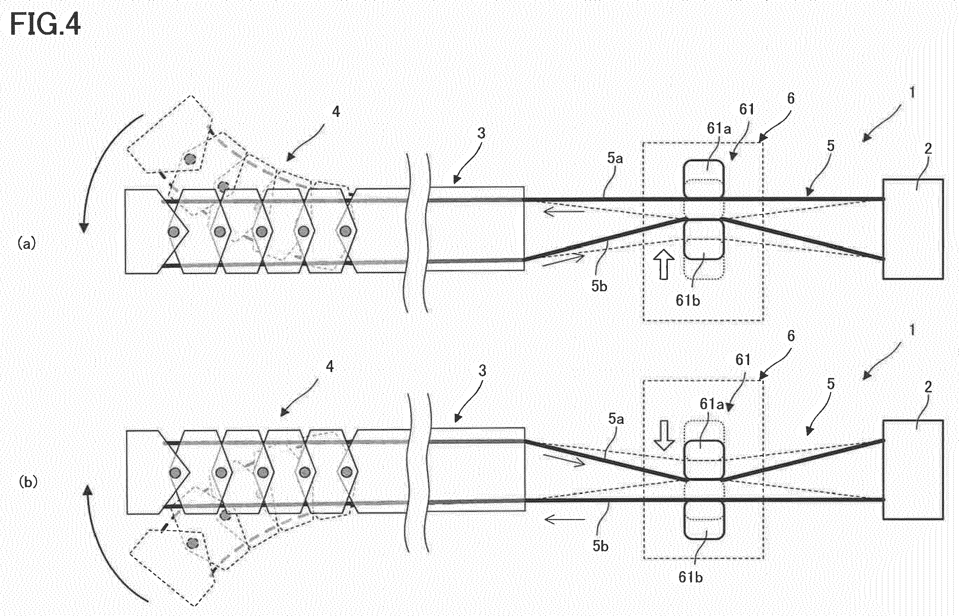

Fig. 4 is illustrative of how the bending assembly 4 in a bent state of the manipulator 1 according to the first embodiment is placed by movement the regulator 6 in a linear state. Fig. 4(a) illustrates that an urge is given to the second coupling member 5b, and Fig. 4(b) illustrates that an urge is given to the first coupling member 5a.

Referring to Fig. 4(a) where it is difficult to operate the bending assembly 4 in a state bent toward the first coupling member 5a by the drive unit 21, movement of the moving member 61 of the regulator 6 toward the first coupling member 5a causes a larger urge to be given by the second moving member 61b to the second coupling member 5b so that the first coupling member 5a comes close to a linear state. In other words, the second coupling member 5b for the bending assembly 4 is pulled by the second moving member 61b so that the first coupling member 5a for the bending assembly 4 is relaxed, and the bending assembly 4 comes close to a linear state.

Referring to Fig. 4(b) where it is difficult to operate the bending assembly 4 in a state bent toward the second coupling member 5b by the drive unit 21, movement of the moving member 61 of the regulator 6 toward the second coupling member 5b causes a larger urge to be given by the first moving member 61a to the first coupling member 5a so that the second coupling member 5b comes close to a linear state. In other words, the first coupling member 5a for the bending assembly 4 is pulled by the first moving member 61a so that the second coupling member 5b for the bending assembly 4 is relaxed, and the bending assembly 4 comes close to a linear state.

Thus, even when the drive unit 21 is not put in operation by reason of power supply shutdown, a malfunction in the drive unit 21 or the like, or even when the power of the drive unit 21 is not transmitted to the coupling member 5, the manipulator 1 according to the first embodiment allows for operation of the moving member 61 of the regulator 6, thereby putting the bending assembly 4 in a bent state into operation.

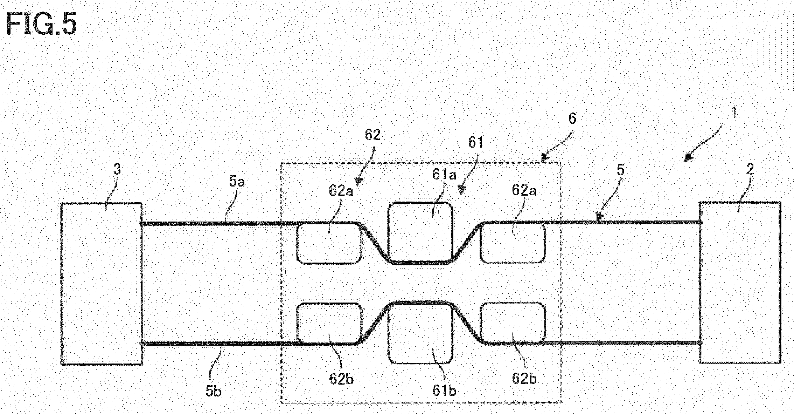

Fig. 5 is illustrative of the regulator 6 in the manipulator 1 according to the second embodiment.

The regulator 6 according to the second embodiment includes a support member 62 on either side of the moving member 61 in addition to the regulator 6 according to the first embodiment. The moving member 61 is movable in a direction intersecting the coupling member 5. In particular, the moving member 61 is preferably movable in a direction orthogonal to the coupling member 5; referring to Fig.5 where the coupling member 5 is urged by the moving member 61 between the support members 62. Preferably, the support members 62 are immobilized or otherwise fixed.

The moving member 61 includes a first moving member 61a adapted to urge the first coupling member 5a and a second moving member 61b adapted to urge the second coupling member 5b. The support member 62 includes a set of second support members 62a mounted on both sides of the first moving member 61a, and a set of first support members 62b mounted on both sides of the second moving member 61b.

The first coupling member 5a is located on one side of a set of first support members 62a and urged by the other surface of the first moving member 61a interposed between a set of the first support members 62a, and the second coupling member 5b is located on the other side of a set of the second support members 62b and urged by one side surface of the second moving member 61b located between a set of the second support members 62b.

If the bending assembly 4 can be bent by the operation unit 23, the moving member 61 is kept against operation so that the coupling member 5 can slide over the surfaces of the moving member 61 and support member 62 to bend the bending assembly 4.

If the bending assembly 4 can hardly be bent by the drive unit 21, for instance, the moving member 61 of the regulator 6 is moved toward the second coupling member 5b. In turn, a larger urge is applied by the first moving member 61a to the first coupling member 5a so that the second coupling member 5b comes close to a linear state. In other words, the first coupling member 5a for the bending assembly 4 is pulled by the first moving member 61a so that the second coupling member 5b for the bending assembly 4 is relaxed. Conversely, as the moving member 61 of the regulator 6 is moved toward the first coupling member 5a, it causes a larger urge to be applied by the second moving member 61b to the second coupling member 5b so that the first coupling member 5a comes close to a linear state.

In the manipulator 1 according to the second embodiment, the provision of the support member 62 in positions adjacent to both sides of the moving member 61 of the regulator 6 causes the coupling member 5 to be urged by the moving member 61 while the coupling member 5 is supported by the support member 62. In other words, the amount of length change in the path taken by the coupling member 5 can be more increased at a shorter moving distance of the moving member 61 than in the case of absence of the support member 62. It is thus possible to make the space for the regulator 6 smaller and the angle of bending of the bending assembly 4 larger.

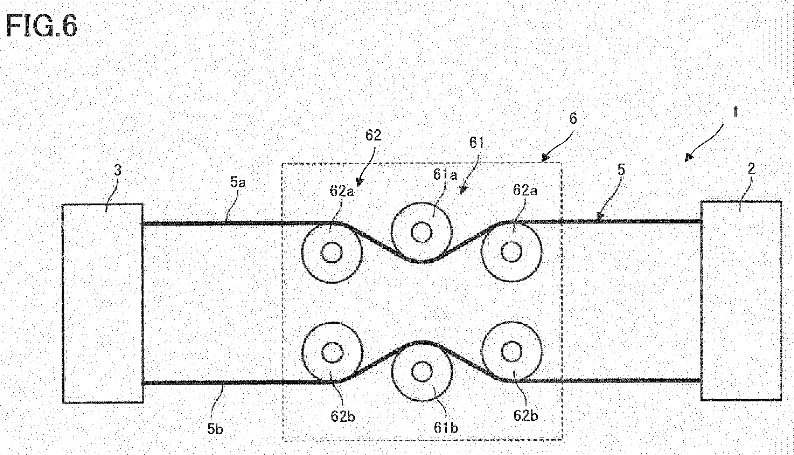

Fig. 6 is illustrative of the regulator 6 in the manipulator 1 according to the third embodiment.

In the regulator 6 of the third embodiment, the moving member 61 and support member 62 in the second embodiment are each defined by a member that is circular and rotatable about an axis, for instance, a pulley. The third embodiment is otherwise the same as the second embodiment, and will not be described anymore.

In the manipulator 1 according to the third embodiment wherein the moving member 61 and support member 62 are each defined by a member that is circular and rotatable about an axis, there is a reduced friction between the coupling member 5 and the moving member 61, the support member 62, allowing for the coupling member 5 to be pushed toward and pulled out of the bending assembly 4 from the main unit 2 during usual operation. If operation by the drive unit 21 is hard to achieve and there is no choice all but to move the moving member 61 too, there is then a reduced friction between the coupling member 5 and the moving member 61 and support member 62, making pushing and pulling of the coupling member 5 smooth.

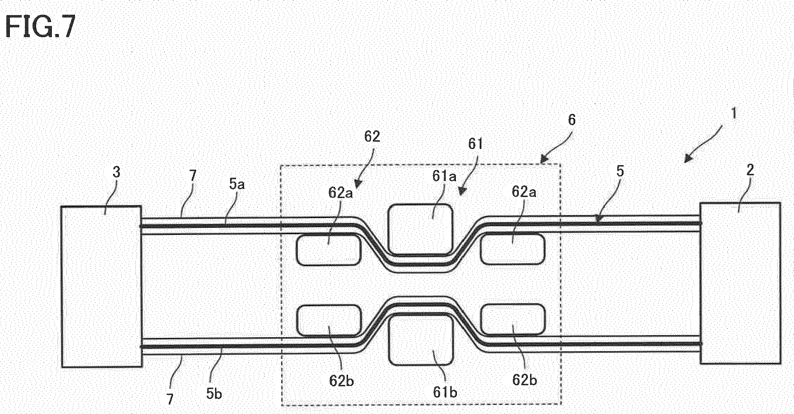

Fig. 7 is illustrative of the regulator 6 in the manipulator 1 according to the fourth embodiment.

The regulator 6 according to the fourth embodiment is configured such that the coupling member 5 according to the second embodiment passes through a sheath 7. The embodiment is otherwise the same as the second embodiment, and will not be explained anymore.

In the manipulator 1 according to the fourth embodiment wherein the coupling member 5 is configured in such a way as to pass through the sheath 7, the coupling member 5 is likely to come in direct contact with the moving member 61 and the support member 62, resulting in a reduced friction between them and making it smooth for the coupling member 5 to be pushed toward and pulled out of the bending assembly 4 from the main unit 2 during normal operation. If operation by the drive unit 21 is hard to achieve and there is no choice all but to move the moving member 61 too, the coupling member 5 is unlikely to come in direct contact with the moving member 61 and the support member 62, resulting in a reduced friction between them and making pushing and pulling of the coupling member 5 smooth.

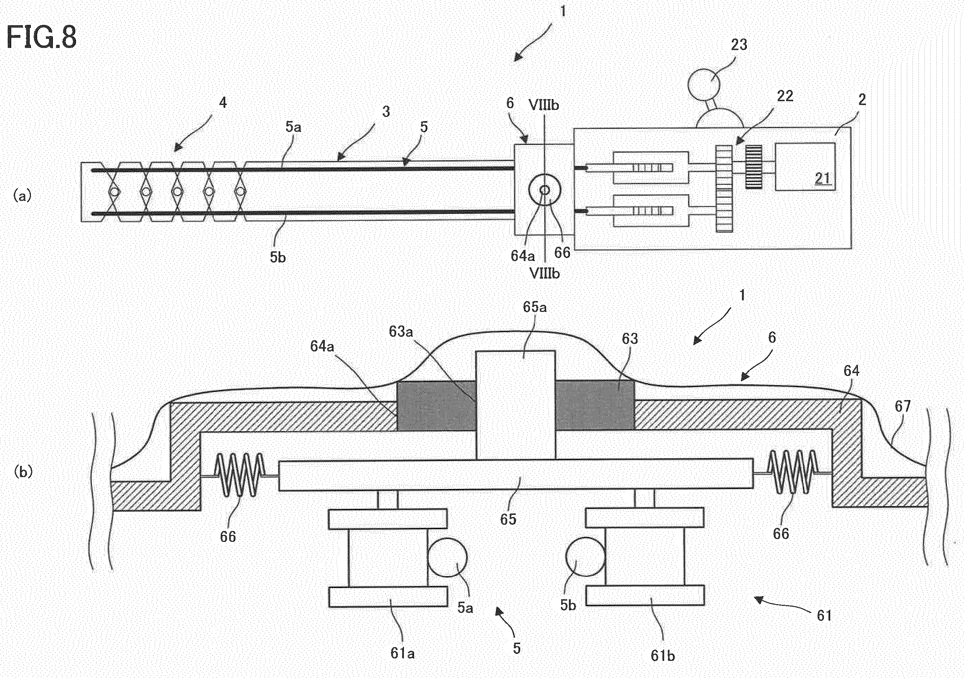

Fig. 8 is illustrative of the regulator 6 in the manipulator 1 according to the fifth embodiment: Fig. 8(a) is a general view of the manipulator 1 according to the fifth embodiment and Fig. 8(b) is a sectional view of Fig. 8(a) as taken on section VIIIb-VIIIb.

The regulator 6 according to the fifth embodiment includes a moving member 61, a stopper 63, a casing 64 adapted to support the stopper 63 during normal operation, a regulating member 65 supported on the casing 64, a resilient member 66 interposed between the casing 64 and the regulating member 65, and a covering 67 adapted to cover all over the regulator 6. The regulator 6 according to the fifth embodiment has a structure in which there is the stopper 63 provided for the purpose of restricting the movement of the moving member 61 during normal operation. Note here that the covering 67 is not necessarily provided.

The moving member 61 is rotatably supported on the regulating member 65, and the regulating member 65 is supported on the casing 64 via the resilient member 66. The stopper 63 is defined by an annular member having an opening 63a in its center. The stopper 63 is fitted into an opening 64a formed in the casing 64, and a regulating portion 65a of the regulating member 65 is fitted in the opening 63a in the stopper 63.

In the absence of the stopper 63, the regulating members 65 and the moving member 61 are free to move with respect to the casing 64. Usually when the regulator 6 is not in use, the coupling member 5 may be pushed and pulled by the drive unit 21; the moving member 61 coming in contact with the coupling member 5 will move unless fixed by the stopper 63. There will then be a possibility that the pushing/pulling movement of the coupling member 5 may not be transmitted to the bending assembly 4. If the movement of the regulating member 65 is restricted by the stopper 63, it is then possible to make sure unerring transmission of power by the coupling member 5 during normal operation.

If the operation by the drive unit 21 is difficult to achieve and there is no option all but to move the moving member 61, just removal of the stopper 63 out of the casing 64 is needed. In turn, the regulating portion 65a of the regulating member 65 is off the stopper 63, placing the regulating member 65 and the moving members 61 in a movable state.

In the manipulator 1 according to the fifth embodiment, the fitting of the stopper 63 into the casing 64 is all that is needed to achieve unerring transmission of power by the coupling member 5 during normal operation, and when the operation by the drive unit 21 is difficult to achieve, it is easy to take off the stopper 63, placing the regulating member 61 in a movable state.

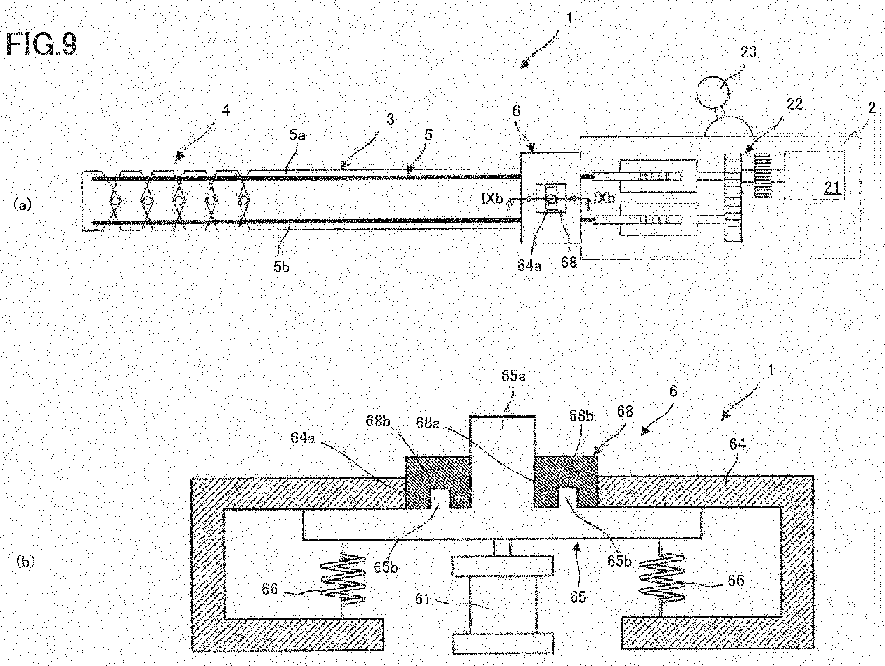

Fig. 9 is illustrative of the regulator 6 in the manipulator 1 according to the sixth embodiment: Fig. 9(a) is a general view of the manipulator 1 according to the sixth embodiment and Fig. 9(b) is a sectional view of Fig. 9(a) as taken on section IXb-IXb.

The regulator 6 according to the sixth embodiment includes a stopper 68, a casing 64 to which the stopper 68 is fixed, a regulating member 65 supported on the casing 64, a resilient member 66 interposed between the casing 64 and the regulating member 65, a covering 67 (not shown) adapted to cover all over the regulator 6, and a moving member 61 that is rotatably supported on the regulating member 65. The regulator 6 according to the sixth embodiment has a structure wherein the stopper 68 different from that in the 5^{th} embodiment is provided to restrict movement of the moving member 61 during normal operation.

The stopper 68 is provided with an opening 68a, below which there is a recess 68b formed. The stopper 68 is fixed to an opening 64a formed in the casing 64. The regulating member 65 is provided in its upper position with a regulating portion 65a and a projection 65b. Usually, the regulating portion 65a is fitted in the opening 68a in the stopper 68, and the projection 65b is fitted in the recess 68b in the stopper 68.

The regulating member 65 is supported on the casing 64 via the resilient member 66. During normal operation, the resilient member 66 is biased or energized in a direction in which the projection 65b of the regulating member 65 is fitted in the opening 68a in the stopper 68.

In a usual state where the regulator 6 is not in use and the coupling member 5 is pushed and pulled by the drive unit 21, the moving member 61 coming in contact with the coupling member 5 will move unless the regulating member 65 is fixed by the stopper 68 in place, and there is a possibility that the pushing/pulling operation of the coupling member 5 may not be transmitted to the bending assembly 4. Thus, if the movement of the regulating member 65 is restricted by the stopper 68, it is then possible to make sure unerring transmission of power by the coupling member 5 during normal operation.

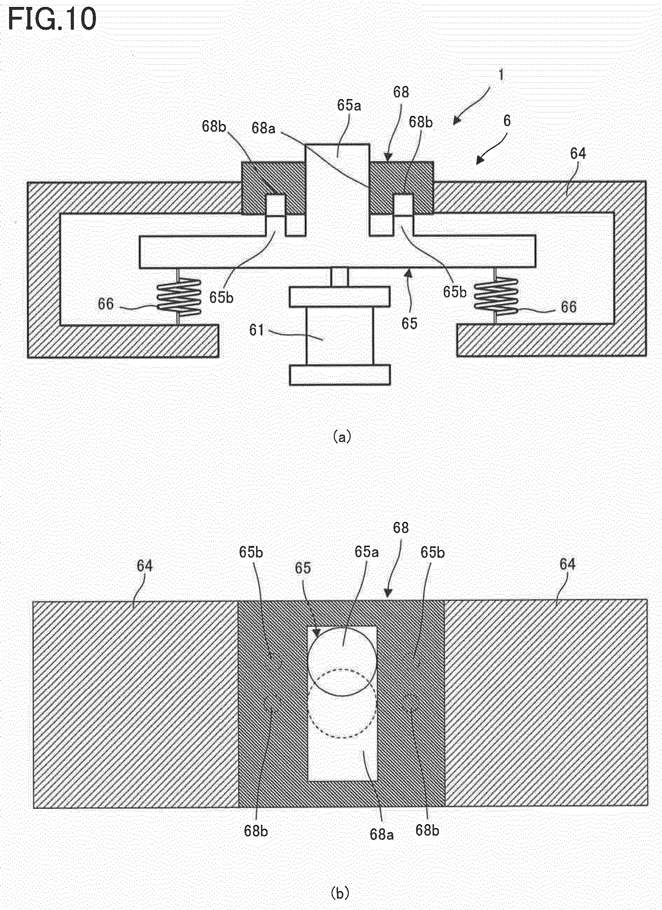

Fig. 10 is illustrative of the regulator 6 in the manipulator 1 according to the sixth embodiment that is placed in a movable state: Fig. 10(a) is a sectional view of the regulator 6 in a movable state corresponding to Fig. 9(b) as taken on a certain section and Fig. 10(b) is illustrative of the regulator 6 in a certain moving state as viewed from above.

If the operation by the drive unit 21 is difficult to achieve and there is a choice all but to move the moving member 61, it is just needed to depress down the regulating portion 65a of the regulating member 65 from above to disengage the projection 65b out of the recess 68b in the stopper 68 and slide the regulating portion 65a while pushing it down. Disengagement of the projection 65b of the regulating member 65 out of the recess 68b in the stopper 68 makes the regulating member 65 and the moving member 61 movable.

According to the manipulator 1 of the sixth embodiment, the fitting of the projection 65b of the regulating member 65 in the recess 68b in the stopper 68 makes sure unerring transmission of power by the coupling member 5 during normal operation, and if the operation by the drive unit 21 is difficult to achieve, it is then possible to take off the stopper 68 easily in one action to place the regulating member 65 in a movable state.

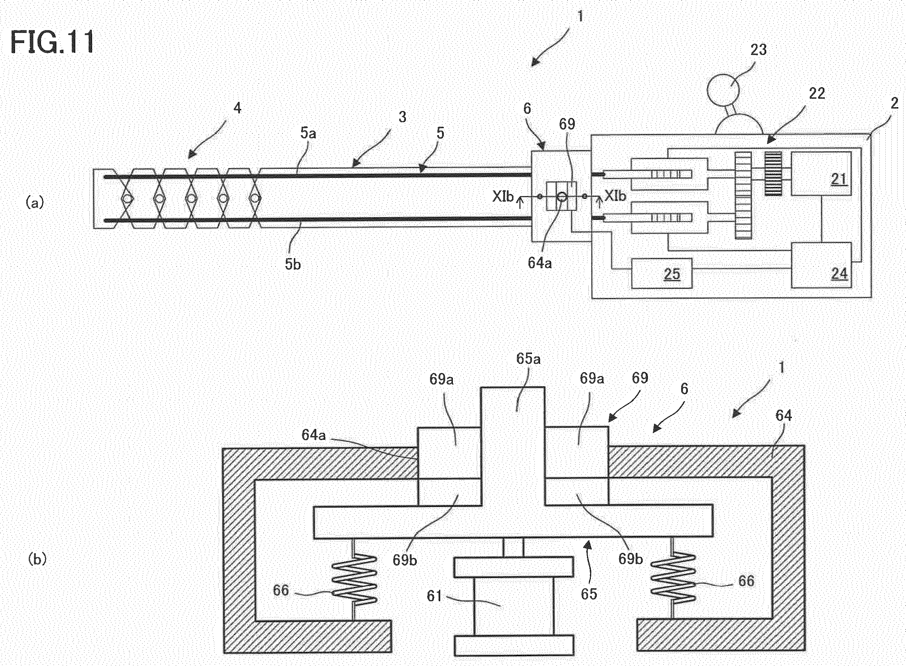

Fig. 11 is illustrative of the regulator 6 in the manipulator 1 according to the seventh embodiment: Fig. 11(a) is a general view of the manipulator 1 according to the seventh embodiment and Fig. 11(b) is a sectional view of Fig. 11(a) as taken on section XIb-XIb.

The manipulator 1 according to the seventh embodiment includes a malfunction detector 24 adapted to detect that the drive unit 21 or power transmission 22 has difficulty in operating, and a control unit 25 adapted to control the regulator 6 in association with the result of detection from the malfunction detector 24.

The regulator 6 according to the seventh embodiment includes a stopper 69, a casing 64, a regulating member 65 supported on the casing 64, a resilient member 66 interposed between the casing 64 and the regulating member 65, a covering 67 adapted to cover all over the regulator 6, and a moving member 61 rotatably supported on the regulating member 65. The regulator 6 according to the seventh embodiment has a structure of restricting movement of the moving member 61 during normal operation.

The stopper 69 includes a disengagement mechanism such an electromagnetic clutch, and includes a first member 69a fixed to the casing 64 and a second member 69b that is movable with respect to the first member 69a.

The regulating member 65 is supported on the casing 64 via the resilient member 66 that is biased or energized such that the regulating portion 65a of the regulating member 65 is fitted in the stopper 69.

With the regulator 6 not in use, the coupling member 5 is usually pushed and pulled by the drive unit 21; the moving member 61 coming into contact with the coupling member 5 will move unless the regulating member 65 remains fixed by the stopper 68, and there is a possibility that the pushing/pulling operation of the coupling member 5 may not be transmitted to the bending assembly 4. Thus, if the movement of the regulating member 65 is restricted by the stopper 69, it is then possible to make sure unerring transmission of power by the coupling member 5 during normal operation.

As the operation by the drive unit 21 gets difficult, it allows for the malfunction detector 24 to detect malfunction so that the control unit 25 performs control such that the stopper 69 is unlocked to place the regulating member 65 and the moving member 61 in a movable state.

According the manipulator 1 of the seventh embodiment, the stopper 69 can be locked to make sure unerring transmission of power by the coupling member 5 during normal operation, and if there is a malfunction detected by the malfunction detector 24, the control unit 25 operates to unlock the stopper 69, placing the regulating member 65 and the moving member 61 in a movable state.

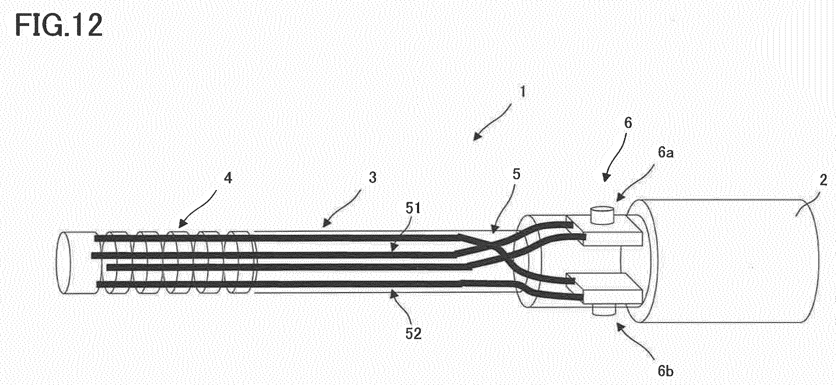

Fig. 12 is illustrative of another example of the manipulator 1 according to the embodiment described here.

The manipulator 1 shown in Fig. 12 is configured such that the bending assembly 4 is bent in a first direction as well as in a second direction orthogonal to the first direction. Specifically, the manipulator 1 here includes a set of coupling members 51 in the first direction, a set of coupling members 52 in the second direction, a first regulator 6a for regulating the coupling members 51 in the first direction, and a second regulator 6b for regulating the coupling members 52 in the second direction. Note here that the first regulator 6a and the second regulator 6b may be selected from those referred to in the aforesaid embodiments.

In such configuration, the bending assembly 4 may be bent by the regulator 6 in various directions.

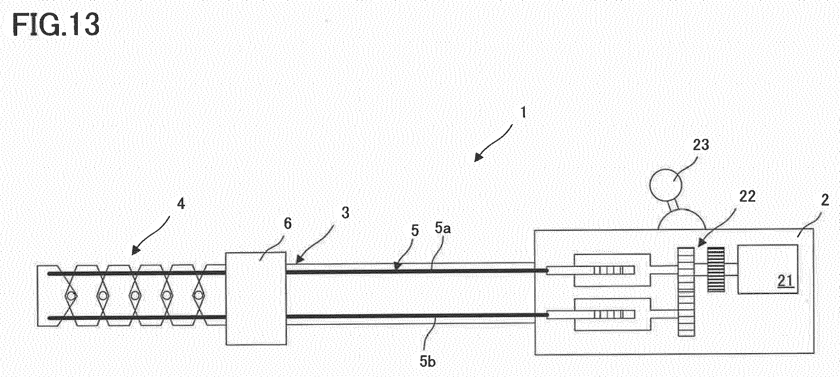

Fig. 13 is illustrative of yet another example of the manipulator 1 according to the embodiment here.

In the manipulator 1 shown in Fig. 13, the elongated member 3 is longer than those in the aforesaid embodiments, and the regulator 6 is positioned away from the main unit 2 and located on the bending assembly 4 side of the elongated member 3. By such position changing of the regulator 6 it is possible to make the degree of freedom in design high.

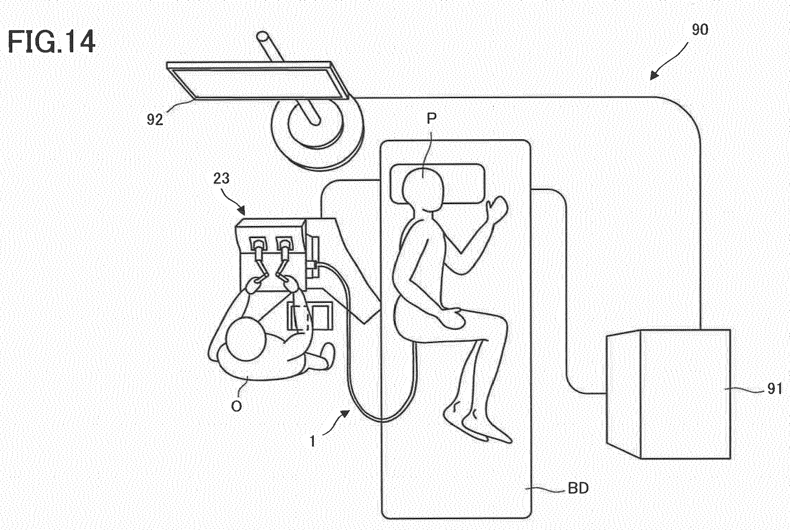

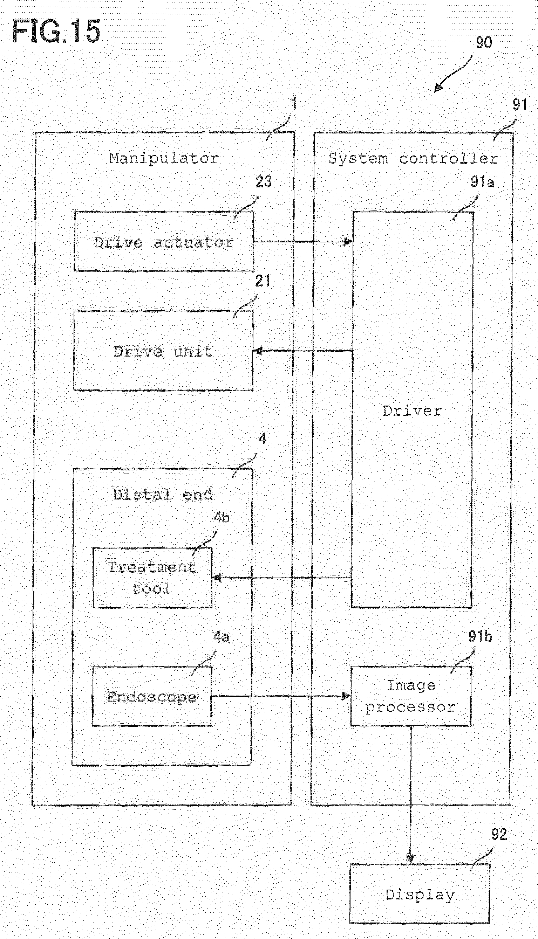

Fig. 14 is illustrative of a manipulator system 90 to which the manipulator 1 according to any one of the aforesaid embodiments is applied, and Fig. 15 is illustrative in architecture of the manipulator system 90 to which the manipulator 1 according to any one of the aforesaid embodiments is applied.

In the embodiment here, the manipulator system 90 incorporates the manipulator 1 according to any one of the aforesaid embodiments. The manipulator system 90 includes a manipulator 1 including a operation unit 23 that is operated by an operator O, a elongated member 3 that is capable of being inserted in the body of a patient P lying down on an operating table BD, for instance, a soft organ such as the large intestine, as shown in Fig. 1, a manipulator 1 including a bending assembly 4 that includes an endoscope attached to the distal end of the elongated member 3 and so on as shown in Fig. 1, a control unit 91 adapted to control the manipulator 1, and a display 92 adapted to display images acquired through the manipulator 1.

As shown in Fig. 14, the operation unit 23 includes a pair of operating handles attached to an actuator table, a footswitch located on the floor surface, and so on. The operation unit 23 may have a multijoint structure. The operation unit 23 is mechanically connected to the elongated member 3 and bending assembly 4 to bend the bending assembly 4. The angle of the operation unit 23 in operation is acquired from an angle acquisition means such as an encoder, and the control unit 91 puts the bending assembly 4 through a driver 91a in operation on the basis of the obtained signals.

The manipulator 1 includes an endoscope, treatment tools or the like at the bending assembly 4. The endoscope includes a viewing/lighting optical system adapted to light the interior of the body to acquire images, an imaging device, and so on. Images gained by the imaging device through the viewing optical system are produced out to an image processor 91b in the control unit 91. The images processed in the image processor 91b are displayed on the display 92, and the operator O operates the manipulator 1 while viewing images appearing on the display 92.

According to such manipulator system 90, even when there is power supply shutdown in the manipulator 1 of Fig. 1 or the operation unit 21 does not work by reason of its malfunction or the like, or even when the power of the operation unit 21 is not transmitted to the coupling member 5, it is possible to move the moving member 61 in the regulator 6 thereby putting the bending assembly 4 in operation.

The manipulator 1 according to the embodiment here includes the main unit 2, the elongated member 3 extending from the main unit 2, the bending assembly 4 connected to the elongated member 3, the coupling member 5 for transmission of power for putting the bending assembly 4 into operation, the operation unit 21 for generating power for the coupling member 4, and the regulator 6 for urging the coupling member 5 in a direction intersecting the path taken by the coupling member 5, making sure unerring regulation of the bending assembly 4 even under abnormal conditions where the power from the operation unit 21 is not transmitted to the bending assembly 4.

According to the manipulator 1 described here, the regulator 6 includes the moving member 61 for urging the coupling member 5 in a direction intersecting the path taken by the coupling member 5, making the angle of bending of the bending assembly 4 larger at a shorter moving distance of the moving member 61.

According to the manipulator 1 described herein, the moving member 61 has given an urge to the coupling member 5, ensuring that the angle of bending of the bending assembly 4 grows larger at a shorter moving distance of the moving member 61.

According to the manipulator 1 described here, the coupling member 5 includes the first coupling members 5a for bending the bending assembly 4 in one direction and the second coupling members 5b for bending the bending assembly 4 in a direction different from the direction of bending the bending assembly 4 by the first coupling members 5a, making the bending assembly 4 bendable in different directions.

According to the manipulator 1 described here, the moving member 61 includes the first moving member 61a that urges the first coupling member 5a in a direction intersecting the path by the first coupling member 5a and the second moving member 61b that urges the second coupling member 5b in a direction intersecting the path taken by the second coupling member 5b, and the regulator 6 includes the regulating portion 65a that moves the first moving member 61a and the second moving member 61b in the same direction, making sure unerring bending of the bending assembly 4 because it gives a pull to the inner moving member where the bending assembly 4 is bent in one operation of the regulating portion 65a to relax the outer moving member.

According to the manipulator 1 described here, the first moving member 61a has given an urge to the first coupling member 5a and the second moving member 61b has given an urge to the second coupling member 5b in a direction opposite to the direction of urging the first coupling member 5a, ensuring that the angle of bending of the bending assembly 4 grows larger at a shorter moving distance of the moving member 61.

According to the manipulator 1 described here, the regulator 6 includes the support member 62 designed such that the moving member 61 urges the coupling member 5 thereby coming in contact with the coupling member 5 from a direction opposite to that of the moving member 61, ensuring that the amount of change in the length of the path taken by the coupling member 5 grows larger at a shorter moving distance of the moving member 61. It is thus possible to reduce the space for the regulator 6 and increase the angle of bending of the bending assembly 4.

According to the manipulator 1 described herein, the support member 62 includes the first support member 62a designed such that the first moving member 61a urges the coupling member 5a thereby coming in contact with the first coupling member 5a from a direction opposite to that of the first moving member 61a and the second support member 62b designed such that the second moving member 61b urges the second coupling member 5b thereby coming into contact with the second coupling member 5b from a direction opposite to that of the second moving member 61b, ensuring that the angle of bending of the bending assembly 4 grows larger at a shorter moving distance of the moving member 61.

According to the manipulator 1 described herein, the support member 62 is rotatable about an axis, ensuring that the coupling member 5 can be smoothly pushed and pulled with a reduced friction.

According to the manipulator 1 described here, there is the sheath 7 provided to cover the coupling member 5, ensuring that the coupling member 5 can be smoothly pushed and pulled with a reduced friction.

According to the manipulator 1 described herein, the regulator 6 includes the casing 64, the regulating member 65 supported on the casing 64, the resilient member 66 interposed between the casing 64 and the regulating member 65, and the stoppers 63, 68, 69 that restrict the movement of the regulating member 65 relative to the casing 64, ensuring that the restriction of movement of the regulating member 65 by the stopper 68 allows for unerring transmission of power by the coupling member 5 during normal operation.

According to the manipulator 1 described here, the stopper 63 is capable of supporting the regulating member 65 and being removed from the casing 64, ensuring that the stopper 63 is unlocked easily with a simplified structure.

According to the manipulator 1 described here, the stopper 68 is fixed to the casing 64 to support the regulating member 65 in a movable state, ensuring that the stopper 68 is unlocked easily with a simplified structure.

According to the manipulator 1 described here, the stopper 69 is defined by a disengagement mechanism that supports the regulating member 65 movably relative to the casing 64, ensuring that the stopper 69 is unlocked easily with a simplified structure.

The manipulator system 90 described here includes the manipulator 1 including treatment tools and an endoscope at the bending assembly 4, the image processor 91b for applying image processing to image signals obtained from the endoscope 4 and the display 92 for displaying image signals sent from the image processor 91b, ensuring that even under abnormal conditions where power of the drive unit 21 is not transmitted to the bending assembly 4, it is possible to move the moving member 61 of the regulator 6 thereby putting the bending assembly 4 in operation.

It is here to be appreciated that the invention is in no sense limited to such embodiments as described above. While the explanation of some embodiments embraces numerous specific details for illustration, it would be obvious to those skilled in the art that diverse variations or modifications made thereto are included within the scope of the invention. In other words, illustrative embodiments of the invention are described without excluding generality from the claimed inventions and imposing any limitation thereon.

### Reference Signs List

- 1:: Manipulator
- 2:: Main unit
- 21:: Drive unit
- 22:: Power transmission
- 23:: Operation unit
- 3:: Elongated member
- 4:: Bending assembly
- 5:: Coupling member
- 6:: Regulator
- 61:: Moving member
- 62:: Support member
- 63, 68, 69:: Stoppers
- 64:: Casing
- 65:: Regulating member
- 66:: Resilient member
- 67:: Covering

## Claims

1. A manipulator comprising:
a main unit,
a elongated member that extends from the main unit,
a bending assembly connected to the elongated member,
a coupling member for transmission of power for putting the bending assembly into operation,
a drive unit for generating power for the coupling member, and
a regulator that is interposed between the bending assembly and the drive unit and urges the coupling member in a direction of intersecting a path taken by the coupling member.

2. The manipulator according to claim 1, wherein:
the regulator includes a moving member that urges the coupling member in a direction of intersecting the path taken by the coupling member.

3. The manipulator according to claim 2, wherein:
the moving member has given an urge to the coupling member.

4. The manipulator according to claim 2 or 3, wherein:
the coupling member comprises a first coupling member that bends the bending assembly in one direction, and a second coupling member that bends the bending assembly in a direction different from a direction in which the bending assembly is bent by the first coupling member.

5. The manipulator according to claim 4, wherein:
the moving member comprises:
a first moving member that urges the first coupling member in a direction of intersecting a path taken by the first coupling member, and
a second moving member that urges the second coupling member in a direction of intersecting a path taken by the second coupling member, and
the regulator comprises a regulating portion that moves the first moving member and the second moving member in one operation in the same direction.

6. The manipulator according to claim 5, wherein:
the first moving member has given an urge to the first coupling member, and
the second moving member has given an urge to the second coupling member in a direction opposite to a direction in which the first coupling member is urged.

7. The manipulator according to claim 5 or 6, wherein:
the regulator includes a support member designed such that the moving member urges the coupling member thereby bringing the support member in contact with the coupling member from a direction opposite to that of the moving member.

8. The manipulator according to claim 7, wherein the support member comprises:
a first support member designed such that the first moving member urges the first coupling member thereby bringing the first support member in contact with the first coupling member from a direction opposite to that of the first moving member, and
a second support member designed such that the second moving member urges the second coupling member thereby bringing the second support member in contact with the second coupling member from a direction opposite to that of the second moving member.

9. The manipulator according to claim 7 or 8, wherein the support member is rotatable about an axis.

10. The manipulator according to any one of claims 1 to 9, which includes a sheath that covers the coupling member.

11. The manipulator according to any one of claims 1 to 10, wherein the regulator comprises:
a casing,
a regulating member supported on the casing,
a resilient member interposed between the casing and the regulating member, and
a stopper that restricts movement of the regulating member relative to the casing.

12. The manipulator according to claim 11, wherein:
the stopper can support the regulating member and be removed from the casing.

13. The manipulator according to claim 11, wherein:
the stopper is fixed to the casing, and movably supports the regulating member.

14. The manipulator according to claim 11, wherein:
the stopper comprises a disengagement mechanism that supports the regulating member movably relative to the casing.

15. A manipulator system comprising:
a manipulator according to any one of claims 1 to 14 and including a treatment tool and an endoscope at the bending assembly,
an image processor for applying image processing to an image signal obtained through the endoscope, and
a display for displaying an image signal sent out from the image processor.
